# FASCICULE DE BREVET EUROPEEN

(11) **EP 3 027 064 B1**
(45) Date de publication et mention de la délivrance du brevet: **26.02.2020**
(21) Numéro de dépôt: 14727605.9
(22) Date de dépôt: 30.04.2014
(51) Int. Cl.: A23L 33/17, A23L 2/44, A23L 2/60, A23L 33/10, A61K 31/375, A61K 31/522, A61K 31/616, A61K 31/198, A61K 9/08, A23L 2/52, A61K 36/77, A61K 9/00, A61K 47/26, A61K 31/7028, A61K 36/258, A23L 33/175, A23L 2/56, A23L 29/30, A23L 27/00, A23L 27/30

(54) **COMPOSITION PHARMACEUTIQUE, DIÉTÉTIQUE OU ALIMENTAIRE LIQUIDE OU SEMI-LIQUIDE DÉPOURVUE D'AMERTUME CONTENANT UN SEL D'ARGININE**
FLÜSSIGE ODER HALBFLÜSSIGE PHARMAZEUTISCHE ODER DIÄTETISCHE NAHRUNGSMITTELZUSAMMENSETZUNG OHNE BITTEREN GESCHMACK UND MIT EINEM ARGININSALZ
PHARMACEUTICAL OR DIETETIC OR FOOD COMPOSITION LIQUID OR SEMI LIQUID COMPRISING ARGININE SALT DEPRIVED OF BITTERNESS

(30) Priorité: 02.05.2013 FR 1301018; 29.10.2013 FR 1360544
(43) Date de publication de la demande: 08.06.2016
(73) Titulaire: Gorny, Philippe, 92410 Ville d'Avray (FR)
(72) Inventeur: Gorny, Philippe, 92410 Ville d'Avray (FR)
(74) Mandataire: Santarelli
(86) Numéro de dépôt international: PCT/FR2014/051041
(87) Numéro de publication internationale: WO 2014/177813

(56) Documents cités:
- EP-A1- 0 511 587
- WO-A1-03/088755
- WO-A1-2011/161655
- GB-A- 2 490 840

## Description

L'invention concerne de nouvelles compositions pharmaceutiques, diététiques ou alimentaires permettant de masquer totalement l'amertume de préparations liquides ou semi-liquides contenant des doses de sels d'arginine égales ou supérieures à un gramme. Le masquage complet de l'amertume rend les compositions de l'invention directement buvables, sans addition d'eau. Elles facilitent la prise, en une fois ou deux fois maximum par jour, de doses élevées d'arginine telles qu'elles sont recommandées pour certaines applications. Nous n'avons pas eu connaissance à ce jour de formulations liquides ou semi-liquides semblables, commercialisées ou appartenant à l'art antérieur.

Par « composition pharmaceutique », nous entendons des formulations dont la ou les substances actives et leurs excipients sont conformes aux exigences réglementaires requises pour les médicaments. Par « composition diététique, » nous entendons des formulations dont la ou les substances actives et leurs excipients sont conformes aux exigences réglementaires requises pour les compléments alimentaires.

Par « composition alimentaire », on entend les produits adaptés à l'alimentation humaine régis par la législation relative aux denrées alimentaires.

Par « sels d'arginine », nous entendons toute combinaison de l'arginine avec une autre entité chimique (par exemple un autre acide aminé, un minéral, un métabolite) aboutissant, par le truchement d'une ou plusieurs liaisons ioniques, à un composé chimique salifié, facilement dissociable dans l'eau, pouvant intégralement libérer la L-Arginine et cette entité chimique, sans aucune modification de leur structure initiale.

Par « alcool », on entend un composé de formule R-OH, dans lequel R représente un groupe hydrocarboné, de préférence l'éthanol. Les glycols et leurs dérivés n'entrent pas dans la catégorie des alcools utilisables selon l'invention.

Par « alcoolat », on entend un liquide obtenu par distillation d'une macération de substances aromatiques végétales (ou animales) dans un alcool. Ledit alcoolat est donc composé d'alcool et de principes aromatiques dont l'origine est végétale, fruits inclus, ou animale, ledit alcool étant tel que défini plus haut.

Par « liquide ou semi-liquide », on entend que ladite composition se trouve sous forme de fluide qui peut être avalé, notamment sans adjonction d'eau, tel que, par exemple une solution, une suspension, une émulsion, une pâte fluide ou un gel.

En particulier, la viscosité de ladite composition est telle qu'elle puisse s'écouler librement de son contenant par simple inclinaison dudit contenant dans la bouche ou dans un récipient. Notamment, la viscosité de ladite composition peut être inférieure ou égale à 3000 mPa.s, plus généralement inférieure ou égale à 200 mPa.s, voire même inférieure ou égale à 50 mPa.s, à température ambiante.

Tandis que les acides aminés dextrogyres ont un goût sucré, les acides aminés lévogyres (naturels) ont, pour au moins huit d'entre eux dont la L-arginine, un goût très amer qui persiste quand la L-arginine est salifiée. Parallèlement, il est notoire que l'emploi d'édulcorants et de substances aromatiques ne parvient pas à supprimer totalement cette amertume (voir par exemple US 2010/0119692A1, paragraphes 0035 et 0036).

L'Arginine est douée de nombreuses vertus thérapeutiques et stimulantes qui justifient sa large utilisation chez l'homme, dans différents domaines : états de fatigue, stimulation sexuelle, accélération des processus de cicatrisation, amélioration de la performance à l'effort chez les coronariens, les insuffisants cardiaques et les transplantés cardiaques, stimulation de l'immunité, pour ne citer que ces quelques exemples.
Ces applications reposent sur des effets biologiques connus de l'arginine et identifiés de longue date : stimulation de l'hormone de croissance (Alba-Roth, J Clin Endocrinol Metab, 1988, 67 (6): 1186-9 - Mateinni et al. Bull Soc Ital Biol Exp 1980. 56: 2254-61)*.* Stimulation d'hormones telles que l'insuline, le facteur de croissance de l'insuline (IGF-1), la prolactine. Stimulation de la synthèse des protéines cellulaires (Barbul, J Parenter Enterai Nutr. 1986; 10(2):227-238)*.* Relaxation du corps caverneux érectile et des fibres musculaires lisses vasculaires via la production de monoxyde d'azote, vasodilatateur puissant dont l'arginine est la seule source naturelle chez l'homme [voir notamment Palmer et coll. Biochem. Biophys. Res. Commun. 153:1251-1256, 1988*b* - Moncada, et coll. Biochem. Pharmacol. 38:1709-1715, 1989]*.* La L-arginine est aussi le précurseur de molécules importantes en physiologie cellulaire comme le glutamate, la créatine ou encore la citrulline (Wu G et coll Biochem J 1998, 336(Pt 1):1-17)*.*

Tant que la dose d'arginine est faible, l'usage de capsules ou de comprimés à avaler reste la modalité la plus pratique, qui de surcroît, évite le contact en bouche du produit avec les papilles gustatives de la langue. Malheureusement, dans de nombreux cas, la dose d'arginine utile est égale ou supérieure à trois grammes (3,00 g) par jour et peut même dépasser 10 grammes (10,0 g) pendant plusieurs jours consécutifs, semaines ou même mois. Dans certaines indications, la dose utile est recommandée en une seule prise, pour des raisons d'observance ou pour la recherche d'un effet particulier à court terme (exemple : stimulation de la fonction sexuelle à la demande). Alternativement, la dose utile gagne en efficacité si elle est administrée en une fois, à savoir 3 grammes, 4 grammes, 5 grammes, 8 grammes, voire 10 grammes d'arginine ou plus. Parfois ces posologies doivent être administrées deux fois par jour. Pour de telles doses quotidiennes, l'usage de la capsule ou du comprimé est fastidieux et inconfortable, obligeant à des prises très répétées si les capsules ou les comprimés contiennent une dose de l'ordre de 0,5 gramme par unité, présentation la plus courante, ou à des prises certes un peu moins nombreuses quand les capsules ou comprimés contiennent une dose d'un gramme, mais avec l'inconvénient d'un volume important, pouvant rendre l'absorption difficile, voire pénible au passage de la capsule ou du comprimé dans la gorge.

Une forme liquide est, dans ce cas, plus pratique. Malheureusement, la solubilité de la L-arginine dans l'eau est faible, 10 fois inférieure en moyenne à celle des sels d'arginine. L'Arginine salifiée est donc préférable quand la modalité liquide est choisie, même si sous cette forme l'arginine réellement administrée ne représente qu'une fraction du total de chaque prise. Par exemple, dans un gramme d'aspartate d'arginine, la quantité véritable de l'arginine n'est que de 560 mg et non de 1000 mg. Ainsi faut-il, pour atteindre une quantité d'un gramme d'arginine, presque doubler la dose de sel administré, qu'il s'agisse d'une forme liquide ou de microgranules solubles dans l'eau.

Plus la quantité de liquide à boire augmente, plus le contact avec les récepteurs du goût de la langue est long et plus a mise au point d'une préparation liquide sans amertume se justifie.

Il existe des formulations buvables à base de sels d'arginine qui facilitent l'absorption de doses égales ou supérieures à 1 gramme comme, par exemple, celles commercialisées en Europe sous le nom de Sargenor® (L-arginine aspartate), Dynamisan® (L-arginine glutamate) ou encore Pargine® (L-arginine aspartate). Ces formulations sont présentées en ampoules buvables à diluer dans de l'eau, à la dose respective de 1 ou 1.5 grammes (Sargenor®), 3 grammes (Dynamisan®) et 5 grammes (Pargine®). Il existe aussi des comprimés effervescents contenant 1 gramme et 1,5 grammes (Sargenor®) de sel d'arginine à dissoudre dans l'eau et même une suspension buvable contenant une dose de 3 grammes de sel d'arginine (Dynamisan®). Cependant, aucune de ces préparations, toutes très anciennes, n'est satisfaisante sur le plan du masquage de l'amertume : celle-ci bien qu'atténuée par les divers excipients employés et par la dilution de la formulation dans l'eau, ne disparaît pas totalement. Les compositions de ces produits sont, à titre d'exemples, décrites ci-dessous :
• Formulation à 5 grammes : L-aspartate de L-arginine ................... 5,00 g q.s.p. 10 ml, pour une ampoule.
Excipients : glycérol, p-hydroxybenzoate de méthyle, p-hydroxybenzoate de propyle, arôme caramel (1), arôme abricot (2), glycyrrhizinate mono-ammoniacal, saccharine sodique, eau potable
   (1) arôme caramel : solution aqueuse concentrée de produits obtenus par chauffage de saccharose ou de glucose
   (2) arôme abricot : vanilline, aldéhydes C18 et C14, acétate d'amyle, caproate d'allyle, ionone, radisol, essences de coriandre, citron, camomille
Indication : retard de croissance
Posologie : une à trois ampoules par jour.

• Formulation à 3 grammes : Glutamate d'arginine.....................3,00 g q.s.p. 10 ml, pour une ampoule
Excipients : sorbitol à 70 % (non cristallisable), saccharine sodique, eau purifiée.
Arôme : fruit de la Passion (voir détail).
Conservateurs : parahydroxybenzoate de méthyle, parahydroxybenzoate de propyle.
Détail fruit de la passion : Alcoolat d'orange, alcoolat de pomme, gamma décalactone, caproate d'allyle, acide 2-méthylbutyrique, delta décalactone, huile essentielle de géranium, lactate d'éthyle, propionate de géranyle, caproate de méthyle.
Indication : asthénie, fonctionnelle, surmenage
Posologie : une ampoule par jour.

• Glutamate d'arginine...................3, 00 g, sachet.
Excipients : acide citrique anhydre, bicarbonate de sodium, sorbitol, silice colloïdale anhydre, arôme pamplemousse/orange (huiles essentielles d'orange et de pamplemousse, jus concentré d'orange, aldéhyde acétique, éthylbutyrate, citral, aldéhyde en C6, linalol, terpinéol, acétate d'éthyle, gomme végétale, maltodextrine, sorbitol), aspartame.
Teneur en sodium : 55 mg/sachet.
La posologie et l'indication sont identiques à la forme liquide précédente.

• Formulations à 1 gramme et 1,5 grammes :
Aspartate d'arginine.............1,00 g, q.s.p. 5 ml, pour une ampoule
Excipients : saccharose (1 g/amp), caramel (E 150), eau purifiée. Conservateurs : parahydroxybenzoates de méthyle (E 218) et de propyle
(E 216). Arôme : abricot (vanilline, aldéhyde benzoïque, acétate d'amyle, diacétyle, ionone, caproate d'allyle, gamma undécalactone, gamma nonalactone, teinture de levisticum, huiles essentielles de citron, d'orange, de bergamote, de coriandre, de néroli, de camomille, de cannelle, de noix de muscade, éthanol, eau purifiée).
Pour un comprimé effervescent :

| | |
|---|---|
| Aspartate d'arginine | 1,5 g |
| Acide ascorbique | 0,5 g |

Excipients : Acide citrique anhydre, bicarbonate de sodium, carbonate de sodium anhydre, citrate de sodium dihydraté, silice colloïdale anhydre, aspartame, saccharine sodique, rouge de betterave (E 162), phosphate sodique de riboflavine, maltodextrine, arôme citron (sorbitol, mannitol, D-glucono-1,5 lactone, huile essentielle de citron).
Indication : état de fatigue passager
Posologie : deux ampoules par jour.

Il convient de noter que les produits décrits ci-dessus sont, en Europe notamment, classés dans la catégorie « médicaments » et que leur formule n'est pas applicable, sur un plan réglementaire, aux compléments alimentaires du fait de certains de leurs excipients jugés trop élevés par les autorités. A l'opposé, les compositions de l'invention peuvent s'appliquer aux deux catégories réglementaires de produits, médicament et complément alimentaire (ou composition diététique), ainsi qu'aux compositions alimentaires liquides contenant ledit complément alimentaire. Enfin, pour toutes les préparations buvables commercialisées sous forme de poudre, granulés ou solutions liquides, dont celles citées plus haut, l'addition d'eau est nécessaire comme indiquée dans leurs notices, tandis que pour les compositions de l'invention, l'addition d'eau n'est pas nécessaire.

Par ailleurs, la demande WO03/088755 décrit un système matriciel de libération d'ingrédients fonctionnels, notamment des acides aminés et en particulier l'arginine non salifiée. Ce système matriciel permet de minimiser ou d'éliminer la dégradation de ces ingrédients.

La demande EP 0 511 587 décrit une composition amincissante contenant un acide aminé en tant qu'accélérateur de la dégradation du glucagon, de préférence l'arginine, éventuellement sous forme de sel, ainsi qu'un dérivé de xanthine et un dérivé de thiamine.

Ces demandes ne décrivent ni ne suggèrent une formulation liquide ou semi-liquide de sels d'arginine à dose élevée, dont la composition permet de masquer l'amertume liée à cette dose.

La nécessité de disposer de doses utiles d'arginine supérieures à un gramme par jour, est illustrée par de nombreuses études : chez des sujets souffrant d'angine de poitrine [Bednarz et al. Int J Cardiol. 2000 15; 75(2-3): 205-10]*,* ou d'insuffisance cardiaque congestive [Hambrecht et al. J Am Coll Cardiol. 2000;1; 35(3): 706-13 - Rector TS et al, Circulation. 199615; 93(12): 2135-41]*,* la prise orale d'au moins 6 grammes d'arginine par jour pendant 6 semaines, améliore la résistance à l'effort physique. Le même résultat a été obtenu chez des patients ayant subi une transplantation cardiaque [Doutreleau S et al. Am J Clin Nutr. 2010 ; 91(5):1261-7] avec 6 grammes d'aspartate d'arginine par jour pendant plusieurs semaines.

Chez des personnes atteintes de claudication intermittente par artérite des membres inférieurs, la dose orale de 8 grammes deux fois par jour pendant 3 semaines, a permis d'augmenter leur périmètre de marche [Boger RH et al. J Am Coll Cardiol. 1998; 32(5): 1336-44]*.* Chez des personnes légèrement hypertendues, des doses orales de 12 grammes d'arginine par jour administrées en trois fois, pendant 4 semaines, ont réduit la pression artérielle de façon significative [Ast J et al. Med Sci Monit. 2010 28;16(5):CR266-71]*.* Chez des femmes ménopausées, des doses orales de 14 grammes par jour pendant 6 mois, ont eu pour effet d'augmenter la force musculaire [Fricke et al. Clin Physiol Funct Imaging. 2008;28(5):307-11]*.* Chez des sujets masculins souffrant de dysfonction sexuelle modérée une préparation contenant 8 grammes d'aspartate d'arginine à prendre en une seule prise à la demande, une à deux heures avant un rapport, a significativement amélioré leur trouble [Neuzillet Y. Andrology. 2013;1(2):223-8 26].

La raison majeure pour laquelle l'usage de l'arginine ne s'est pas généralisé dans ces différentes applications n'est pas liée à une intolérance quelconque du produit, qui est la cause de très peu d'effets indésirables, même à des doses de 9 ou 10 grammes deux fois par jour (Tousoulis et coll, Vasc Med 2002,7, 203*),* mais davantage à l'absence d'une formulation plaisante et pratique, permettant l'absorption de doses conséquentes d'arginine, utiles à ces applications, via une modalité simple, agréable au goût et confortable. Il serait donc souhaitable de disposer de compositions, pour l'administration de sels d'arginine à des doses comprises entre un et dix grammes, en une seule prise voire deux maximum par jour, directement, sans addition d'eau, ce qui en faciliterait l'usage et favoriserait l'observance. Ceci n'est envisageable que si l'amertume de la préparation est totalement masquée. C'est l'objet principal de la présente invention.

Il a donc été recherché de nouvelles préparations liquides ou semi liquides, directement buvables, sans addition d'eau, contenant des doses de sels d'arginine égales ou supérieures à un gramme, et dont l'amertume est totalement masquée. Le masquage complet de l'amertume rendant les compositions de l'invention directement buvables, sans addition d'eau, facilite la prise de doses élevées d'arginine.

De façon surprenante, nous avons découvert qu'une formulation liquide très simple contenant un édulcorant faible, de préférence le maltitol, ainsi que, éventuellement, un alcool ou un alcoolat, de préférence un alcoolat de citron ou de mandarine, et, éventuellement, au moins un édulcorant tel que le sucralose ou l'aspartame, permettait lorsqu'on les combinait ensemble à des ratios spécifiques, de masquer complètement l'amertume de l'arginine administrée sous forme salifiée liquide ou semi-liquide, et ceci quelle qu'en soit la dose.

Sans vouloir être lié par la théorie, on peut émettre l'hypothèse de travail selon laquelle le rapport entre le sel d'arginine et le maltitol joue un rôle important dans l'atténuation de l'amertume, alors qu'un tel effet n'avait jamais été décrit ni suggéré dans la littérature. L'effet obtenu est renforcé en présence d'alcool ou d'un alcoolat plus que par tout autre ajout d'excipient. A la suite de très nombreux essais, il a été découvert qu'un subtil équilibre entre les éléments décrits ci-dessous, permet le masquage complet de l'amertume d'arginine salifiée, donnée sous forme liquide ou semi-liquide, quelle que soit sa dose à partir d'un gramme et plus. Notamment, ces éléments sont les suivants :
- l'ajout de maltitol, qui permet d'épaissir agréablement la préparation tout en étant un édulcorant faible, à un poids au moins égal à celui du sel d'arginine, notamment à raison de 1 à 1,5 fois le poids de l'arginine salifiée dans la préparation ;
- l'ajout éventuel, à la quantité d'eau nécessaire pour solubiliser un sel d'arginine, d'un alcool ou d'un alcoolat, en particulier un alcoolat de citron ou de mandarine, à une dose en poids comprise entre 0,1% et 12%, notamment entre 1% à 5%, et de préférence entre 1% à 3% du poids total de la composition ;
- l'ajout éventuel d'au moins un, de préférence deux édulcorants forts donnés à faible dose chacun, qui permet la disparition complète de l'amertume. A l'opposé, l'utilisation d'un seul de ces édulcorants avec un sel d'arginine seul ou enrichi d'édulcorants autres que ceux de l'invention, même à doses fortes, échoue à masquer l'amertume de l'arginine.
- sous réserve d'augmenter légèrement l'alcoolat (sans dépasser les limites indiquées plus haut), quand le poids du sel d'arginine augmente, il est possible de maintenir les autres additifs de la composition à des taux inférieurs ou conformes à tous les schémas réglementaires autorisés, qu'il s'agisse de médicaments ou de compléments alimentaires, sans inconvénient sur le masquage complet de l'amertume. C'est là un autre aspect de l'invention.

L'invention concerne donc une composition pharmaceutique ou diététique aqueuse, liquide ou semi-liquide, comprenant au moins un sel de L-arginine à raison d'au moins un gramme dudit sel par unité de prise, ladite composition, comprenant
- du maltitol, notamment à raison de 1 à 1,5 fois le poids dudit sel d'arginine dans la composition, et
- de l'eau, à raison d'un poids de 1,5 à 3 fois, notamment de 1,8 à 2, 5 fois le poids dudit sel d'arginine dans la composition.

En particulier, l'invention concerne une composition pharmaceutique ou diététique aqueuse, liquide ou semi-liquide, comprenant au moins un sel de L-arginine à raison d'au moins un gramme dudit sel par unité de prise, ladite composition, comprenant :
- de 15 à 30%, de préférence de 20% à 30% en poids de sel de L-arginine,
- de 15 à 45%, de préférence de 20 à 40%, en particulier de 20 à 30% en poids de maltitol,
par rapport au poids total de la composition.

Le reste de la composition est constitué, en plus de l'eau, par au moins un excipient, notamment au moins un additif conservateur et/ou au moins un antioxydant, et/ou, par exemple, par des composants permettant d'améliorer le masquage de l'amertume ou le goût, notamment un alcool ou un alcoolat tels que mentionnés plus haut, un édulcorant fort par exemple, et/ou, éventuellement, d'autres substances actives que le sel de L-arginine, la totalité des composants n'excédant pas 100% en poids total de la composition.

En particulier, le poids d'eau (potable ou purifiée) est égal à 1,5 fois minimum jusqu'à 3 fois maximum, notamment de 1,8 fois à 2,5 fois, le poids du sel d'arginine de la préparation. Le poids d'eau peut-être compris entre ces deux limites et représenter, par exemple 1,85 fois ou 1,87 fois ou 1,92 fois le poids de l'arginine salifiée,

La quantité d'eau dans la composition peut être, par exemple, de 30 à 60 % en poids, notamment de 30 à 50% en poids par rapport au total de la composition.

On utilisera de l'eau potable ou purifiée, de qualité acceptable sur le plan pharmaceutique ou diététique.

Par « unité de prise », on entend la forme galénique liquide ou semi-liquide ou l'unité de formulation ou de conditionnement de produit diététique ou pharmaceutique contenant une dose de sels d'arginine égale ou supérieure à un gramme qui est absorbée par l'utilisateur/le patient, une ou plusieurs fois, pour obtenir la dose utile.

Avantageusement, ladite composition est une composition pharmaceutique.

Selon un autre aspect, ladite composition est une composition diététique (ou complément alimentaire). Elle peut, dans ce cas, être mélangée à une composition alimentaire liquide ou semi-liquide.

Dans le cas d'une composition alimentaire, la composition diététique décrite ici est, préalablement à son utilisation et à sa commercialisation, mélangée à une boisson gazeuse ou non, ou à une préparation liquide ou semi-liquide de type laitage, crème, gel etc., et donc diluée. Elle contient volontiers alors la quantité maximale de sel d'arginine autorisée par la législation (un gramme ou plus selon les cas). Plusieurs compositions diététiques unitaires peuvent, le cas échéant, être mélangées à une préparation alimentaire liquide ou semi-liquide.

De préférence, ladite composition pharmaceutique ou diététique contient un seul sel de L-arginine.

Avantageusement, ladite composition pharmaceutique ou diététique comprend également de 0,1% à 12%, notamment 1 à 5%, de préférence 1 à 3 % en poids d'un alcool ou d'un alcoolat.

De préférence, ladite composition contient en outre au moins un, de préférence deux édulcorants forts, tels que, par exemple le sucralose ou l'aspartame, par exemple à raison de 0,01 à 1%, notamment de 0,01% à 0,5% en poids par rapport au poids total de la composition.

L'invention concerne également une composition pharmaceutique ou diététique, liquide ou semi-liquide, comprenant un sel de L-arginine à la dose d'au moins un gramme et les composés suivants :
- un poids d'eau purifiée égal à 1,8 fois minimum et 2,5 fois maximum, le poids du sel d'arginine de la préparation ou un poids d'eau compris n'importe où entre ces deux limites,
- un alcoolat, et
- du maltitol.

L'invention concerne, en particulier, une composition pharmaceutique ou diététique aqueuse, liquide ou semi-liquide, comprenant au moins un sel de L-arginine à raison d'au moins un gramme dudit sel par unité de prise, ladite composition comprenant :
- de 20% à 30% en poids de sel de L-arginine,
- de 20% à 40% en poids, de préférence 20% à 30% en poids de maltitol, et
- de 0,1% à 12% en poids, de préférence de 1 à 5% en poids d'un alcool ou d'un alcoolat,
par rapport au poids total de la composition.

Avantageusement, la composition pharmaceutique ou diététique selon l'invention comprend un sel de L-arginine à raison de un à dix grammes, notamment cinq grammes ou huit grammes.

Une composition optimale peut comprendre, à titre d'exemple :
- Un poids d'eau (potable ou purifiée) égal à 1,5 fois minimum jusqu'à 3 fois maximum, notamment de 1,8 fois à 2,5 fois, le poids du sel d'arginine de la préparation. Le poids d'eau peut-être compris entre ces deux limites et représenter, par exemple 1,85 fois ou 1,87 fois ou 1,92 fois le poids de l'arginine salifiée,
- du maltitol à un poids compris entre 1 fois et 1,5 fois le poids de l'arginine salifiée dans la préparation, ou compris entre ces deux limites par exemple égal à 1,25 fois ou 1,30 fois le poids du sel d'arginine,
- Un alcoolat, préférablement de citron ou de mandarine, représentant en poids 1% à 5% et de préférence 1% à 3 % du poids total de la composition,
- du sucralose, par exemple à raison de 0,01% à 1% du poids total de la composition plus particulièrement de 0,01 à 0,5% et, de préférence, d'environ 0,02 % à environ 0,04 % du poids total de ladite composition correspondant alors une teneur allant de 212mg/L à 424 mg/L ou comprise entre ces deux limites réglementaires, et/ou
- de l'aspartame, par exemple à raison de 0,01% à 1% du poids total de la composition, plus particulièrement de 0,01 à 0,5% et, de préférence, d'environ 0,02% à environ 0,08 % du poids total de ladite composition, correspondant alors à une teneur allant de 315mg/kg à 1200 mg/kg ou comprise entre ces limites réglementaires,
- Enfin, au moins un excipient conservateur, notamment l'acide sorbique et le sorbate de potassium ou leurs mélanges, à la dose maximale cumulée de 2000mg/L, et au moins un antioxydant, par exemple l'acide ascorbique, à la dose réglementaire autorisée.
- Les concentrations ci-dessus, quand elles sont exprimées par litre (mg/L), doivent se comprendre par litre de la composition totale. Les concentrations exprimées par kilogramme (mg/Kg) doivent se comprendre par kilogramme de la composition totale. Si par exemple la composition représente un volume total de 0,020 L, la concentration en sucralose pourra varier de 212 x 0,02 = 4,24 mg/L arrondi à 4,2 mg/ml à 424 x 0,02=8,48 arrondi à 8,5 mg/L. Si par exemple la composition représente un poids total de 0,02kg, la concentration en aspartame pourra varier de 315x 0,02 = 6,3 mg/kg à 1200 x 0,02= 24 mg/kg.

Une composition préférée comprend, outre le sel d'arginine, de l'eau potable, un alcool ou un alcoolat, du maltitol et des conservateurs aux doses indiquées plus haut.

En tant qu'alcool, on utilisera de préférence de l'éthanol, à un poids égal à 0,1% à 12%, notamment 1% à 5%, et de préférence 1 à 3%, du poids total de la composition.

Une autre composition de l'invention comprend un sel d'arginine, de l'eau potable, un alcoolat, du maltitol, du sucralose et/ou de l'aspartame, ainsi que des conservateurs aux doses indiquées plus haut.

De manière surprenante, on a trouvé qu'un grand nombre d'édulcorants usuels, utilisés seuls ou combinés, sont peu efficaces pour masquer l'amertume de l'arginine salifiée utilisée à doses égales ou supérieures à un gramme. C'est le cas par exemple de la stevia rebaubania, de l'acesulfame de potassium ou encore du sodium saccharinate. L'aspartame seul, le sucralose seul n'ont qu'un effet d'atténuation très partiel. C'est leur combinaison avec le maltitol et un alcoolat aux quantités indiquées plus haut qui est efficace pour masquer complètement l'amertume de la composition. Certains alcoolats, comme l'alcoolat de menthe, de pomme ou d'orange, bien qu'ils soient forts en arôme et produisent du goût, sont moins performants que les alcoolats de mandarine et citron quand ils sont combinés aux trois édulcorants de la composition optimale ou à d'autres. Les huiles essentielles, plus puissantes encore, sont à elles seules totalement inefficaces. La combinaison d'alcoolats et d'huiles essentielles, sans les autres ingrédients de l'invention, ne change rien au résultat : l'amertume de l'arginine persiste. Par contre, une huile essentielle, par exemple une huile essentielle de citron ou de mandarine, associée à la composition de l'invention, ajoute du goût sans altérer le masquage de l'amertume.

La composition selon l'invention peut également comprendre une ou plusieurs autres substances actives autre(s) qu'un sel de L-arginine, notamment : certaines purines telles que l'adénosine monophosphate (AMP), ainsi que le décrivent les brevets EP1137420 et US6506736, notamment à raison de 100 mg à 1000 mg par unité de prise ; des composés minéraux comme par exemple le magnésium ou le potassium ou l'un de leurs sels ; d'autres acides aminés relativement solubles dans l'eau, comme par exemple la N-acétyl-cystéine, la cystéine, la cystine et la lysine ou encore des métabolites comme la S-adénosyl-méthionine ou des sels de ces diverses entités chimiques. Peuvent être aussi ajoutés aux sels d'arginine de la composition des vitamines (par exemple B1, B6, B12, C), de la caféine ou une plante riche en caféine comme le guarana, ou encore de la maca (Lepidium meyenii) sous forme soluble, ou des extraits solubles de celle-ci, ou encore de l'acide acétylsalicylique, ou enfin des huiles essentielles, notamment de mandarine ou de citron.

La fabrication d'une composition avantageuse selon l'invention peut comprendre les points importants suivants :
- Tout d'abord l'incorporation progressive à température ambiante des matières premières en respectant de 1 à 10 l'ordre suivant: 1) l'eau purifiée - 2) l'acide sorbique - 3) le sorbate de potassium - 4) l'acide ascorbique - 5) le sucralose - 6) l'aspartame - 7) une autre substance active éventuelle, par exemple l'adenosine monophosphate - 8) le sel d'arginine (L-arginine aspartate par exemple) - 9) le maltitol - 10) enfin l'alcoolat (de citron par exemple), le tout étant additionné sous agitation défloculeuse avec contre-pale assurant un cisaillement à vitesse moyenne.

Les étapes 2 à 7 et 10 ne sont pas toutes nécessairement effectuées et dépendent de l'ajout optionnel de ces composés.
- Il convient d'ajuster à la prise de volume les substances versées au fur et à mesure. L'incorporation du sel d'arginine et du maltitol, notamment, doit toujours être réalisée de manière régulière et non d'un coup.
- Au cours de cette manipulation il faut, après ajout d'un composé, attendre que le liquide de la préparation redevienne limpide avant d'introduire la matière première suivante.
- La dissolution de l'acide sorbique peut être réalisée à chaud (40 à 70 degré Celsius) afin d'accélérer le processusL'ajout éventuel de la composition ainsi obtenue à un produit alimentaire liquide ou semi-liquide est réalisée dans un deuxième temps

La composition selon l'invention, en tant que médicament ou composition diététique, peut être administrée par voie orale ou sublinguale. A cet effet, elle peut être présentée sous toute forme permettant ces administrations (solutions ou émulsions buvables, gels, ainsi que des poudres, granulés, tablettes ou comprimés lyophilisés issus de la composition liquide). Ces formes galéniques sont préparées de façon usuelle, conformes aux bonnes pratiques de fabrications (GMP: Good Manufacturing Practice), et peuvent contenir des excipients et véhicules classiques appropriés, autres que ceux appartenant à la présente invention. La forme liquide buvable directement, sans ajout d'eau ni dilution complémentaire en solution aqueuse, est la forme préférée.

La composition alimentaire selon l'invention peut, par exemple, se présenter sous forme liquide à additionner à une boisson existante, mais plus généralement est conditionnée avec ladite boisson ou la préparation semi-liquide (émulsion, mousse, crème existante, etc.) dans un récipient tel qu'une canette, une bouteille, un flacon, un pot, un tube etc dont le contenu peut aller, par exemple, de 50 ml à 2000 ml, notamment de 100 ml à 1500 ml.

L'arginine salifiée peut se présenter sous différentes formes notamment sous forme de chlorhydrate, de glutamate, de pyroglutamate, d'aspartate, de pidolate, de ketoglutarate d'arginine, ou d'autres sels d'arginine, ou leurs mélanges. La composition de l'invention peut être utilisée sur de longues périodes, par exemple en cures de plusieurs semaines plusieurs fois par an, ou en cures très courtes de quelques jours ou encore de façon ponctuelle, intermittente, « à la demande ».

L'invention a également pour objet la composition pharmaceutique ou diététique décrite plus haut, pour son utilisation dans la prévention ou le traitement de différents troubles pathologiques animaux ou humains, en particulier :
- pour prévenir et/ou traiter les troubles de la réponse physiologique et/ou anatomique à la stimulation sexuelle chez les humains ;
- pour prévenir et/ou traiter les affections cardiovasculairesdans lesquelles une relaxation des fibres musculaires lisses du cœur et des vaisseaux est souhaitée ou apporte un bénéfice, telles que, par exemple, l'hypertension artérielle, l'artérite des membres inférieurs, l'insuffisance cardiaque et les cardiopathies ischémiques ;
- pour prévenir et/ou traiter les affections cardiovasculaires dues ou associées à un dysfonctionnement des cellules endothéliales vasculaires, par exemple, l'athérosclérose et/ou le diabète, ou encore conditionnées, par exemple, par l'âge ou l'intoxication tabagique;
- pour stimuler l'hormone de croissance ;
- pour accélérer les processus de cicatrisation ;
- pour prévenir et/ou traiter les états de fatigue.

L'invention concerne également l'utilisation d'une association de maltitol, et d'un alcool ou d'un alcoolat, pour masquer l'amertume d'un composé dans une composition pharmaceutique ou diététique.

L'invention est illustrée de manière non limitative par les exemples ci-dessous.

### EXEMPLE 1 : tests qualitatifs

Des essais ont été effectués sur plusieurs dizaines de préparations évaluées chacune à l'aveugle par un panel de trois experts entrainés.

Les scores sur l'amertume ont été portés de la façon suivante : 1 = non satisfaisant, 2= passable, 3 = satisfaisant (aucune amertume), donnant un score total allant de 3 au minimum à 9 au maximum, suivant la note donnée par chacun.

Quatre autres sujets témoins non entrainés ont ensuite jugé, à l'aveugle, sans addition d'eau, à la fois la qualité du goût et l'absence d'amertume des formulations sélectionnées par les experts comme les plus performantes, ainsi que les formulations commercialisées, en attribuant des notes allant de 1 à 5, ce qui a défini un score total allant de 5 au minimum à 20 au maximum, suivant la note donnée par chacun.
Les résultats sont les suivants :

### Tests des experts

Les essais avec produits commercialisés Dynamisan®, Sargenor® et Pargine® ont été jugés passables par le panel d'experts quand ils sont dilués dans de l'eau (score total de 6 pour les trois produits) et globalement non satisfaisants sans ajout d'eau (score total de 3 pour la Pargine® et de 4 pour les deux autres produits).

Les essais avec les édulcorants suivants : stevia rebaubania, acésulfame de potassium, saccharinate de sodium, aspartame, sucralose, utilisés seuls ou en combinaison dans des préparations avec 8 grammes d'aspartate d'arginine, n'ont abouti qu'à une atténuation très partielle de l'amertume (score total de 3 ou 4 selon les associations avec ou sans addition d'eau).

L'utilisation d'huiles essentielles ayant des goûts de menthe, pomme ou orange, seules ou associées aux édulcorants cités ci-dessus n'a pas amélioré le masquage de l'amertume (pas d'amélioration des scores).

L'utilisation d'alcoolat de citron ou mandarine s'est avérée plus efficace que les huiles essentielles et les édulcorants seuls pour réduire l'amertume de la L-arginine (scores totaux atteignant 5 sans addition d'eau et 6 avec addition d'eau).

L'utilisation de maltitol seul en quantité au moins égale à celle de l'arginine atténue considérablement son amertume, de façon plus efficace que tous les autres édulcorants (score total de 7 sans dilution dans l'eau - score de chaque expert jamais inférieur à 2). Sa combinaison avec un alcoolat de citron ou de mandarine obtient la note maximum de 3 chez deux experts et un score total de 8/9 (sans addition d'eau), pour les deux formulations principales.

### Tests des sujets témoins

Les compositions commercialisées Dynamisan® Sargenor® et Pargine® testées sans addition d'eau, se sont vues attribuer par le panel de témoins les notes de 12, 10 et 8 respectivement. Les compositions sélectionnées par les experts ont toutes atteint un score total égal ou supérieur à 16.

Les 4 compositions ci-dessous (exemples 2 à 5) établies à partir de celles sélectionnées par les experts se sont vus attribuer par le panel des sujets témoins le score total maximum de 20.

### EXEMPLE 2 :

Une composition typique de l'invention associe :

| Tableau 1 | | |
|---|---|---|
| **Formulation goût citron** | **g** | **% en poids** |
| eau | 15 | 44,6 |
| aspartate d'arginine | 8 | 23,8 |
| maltitol | 10 | 29,7 |
| alcoolat de citron | 0,5 | 1,5 |
| sucralose | 0,007 | 0,020 |
| aspartame | 0,019 | 0,056 |
| acide sorbique | 0,02 | 0,06 |
| sorbate de potassium | 0,04 | 0,12 |
| acide ascorbique | 0,02 | 0,06 |
| **Total** | **33,6** | **100** |

Dans cette composition l'alcoolat de citron peut varier de 0,5 gramme à 0,8 gramme et peut-être remplacé par de l'alcoolat de mandarine aux mêmes doses.

Le sucralose est à la dose de 212 mg/L, pesant dans cet exemple 7 mg.

L'aspartame est à la dose de 576 mg/L pesant dans cet exemple 19 mg mais peut-être utilisé à une dose moindre de 350mg/L sans altération du masquage de l'amertume.

L'acide sorbique et le sorbate de potassium sont utilisés à la dose cumulée de 2000 mg/L représentant ici 60 mg du poids total.

### Exemple 3 :

Dans une autre composition typique de l'invention, avec 0,6 gramme d'alcoolat, on ajoute à la préparation précédente de l'adénosine monophosphate (tableau 2).

| Tableau 2 | | |
|---|---|---|
| **Formulation goût citron** | **g** | **% en poids** |
| eau | 15 | 44,24 |
| aspartate d'arginine | 8 | 23,59 |
| adenosine monophosphate (AMP) | 0,2 | 0,59 |
| maltitol | 10 | 29,49 |
| alcoolat de citron | 0,6 | 1,77 |
| sucralose | 0,007 | 0,021 |
| aspartame | 0,019 | 0,056 |
| acide sorbique | 0,02 | 0,06 |
| sorbate de potassium | 0,04 | 0,12 |
| acide ascorbique | 0,02 | 0,06 |
| **Total** | **33,9** | **100** |

### Exemple 4 :

Une autre composition typique de l'invention utilise la même formulation que dans le tableau 1 avec 5 grammes de pyroglutamate d'arginine ou 5 grammes d'aspartate d'arginine.

| Tableau 3 | | |
|---|---|---|
| **Formulation goût citron** | **g** | **% en poids** |
| eau | 10 | 45 |
| pyroglutamate d'arginine ou aspartate d'arginine | 5 | 22,5 |
| maltitol | 6,5 | 29,3 |
| alcoolat de citron | 0,6 | 2,7 |
| sucralose | 0,007 | 0,031 |
| aspartame | 0,019 | 0,085 |
| acide sorbique | 0,02 | 0,09 |
| sorbate de potassium | 0,04 | 0,18 |
| acide ascorbique | 0,02 | 0,09 |
| **Total** | **22,20** | **100** |

### Exemple 5 :

Une autre composition de l'invention utilise la même formulation que dans le tableau 3 avec 10 grammes de pyroglutamate d'arginine ou 10 grammes d'aspartate d'arginine.

| Tableau 4 | | |
|---|---|---|
| **Formulation goût citron** | **g** | **% en poids** |
| eau | 20 | 45 |
| aspartate d'arginine ou pyroglutamate d'arginine | 10 | 22,5 |
| maltitol | 13 | 29,3 |
| alcoolat de citron | 0,12 | 2,7 |
| sucralose | 0,014 | 0,031 |
| aspartame | 0,038 | 0,085 |
| acide sorbique | 0,04 | 0,09 |
| sorbate de potassium | 0,08 | 0,18 |
| acide ascorbique | 0,04 | 0,09 |
| **Total** | **44,40** | **100** |

## Revendications

1. Composition pharmaceutique ou diététique aqueuse, liquide ou semi-liquide, comprenant au moins un sel de L-arginine à raison d'au moins un gramme dudit sel par unité de prise, ladite composition, comprenant :
- du maltitol de préférence à raison de 1 à 1,5 fois le poids dudit sel d'arginine dans la composition, et
- de l'eau, à raison d'un poids de 1,5 à 3 fois le poids dudit sel d'arginine dans la composition, et
- au moins un alcool ou un alcoolat.

2. Composition pharmaceutique ou diététique aqueuse, liquide ou semi-liquide, comprenant au moins un sel de L-arginine à raison d'au moins un gramme dudit sel par unité de prise, ladite composition comprenant :
- de 15 à 30% en poids, de préférence de 20% à 30% en poids de sel de L-arginine ;
- de 15 à 45%, de préférence de 20% à 40% en poids, de maltitol, par rapport au poids total de la composition, et
- de 0,1 à 12% en poids, de préférence de 1 à 5% en poids d'un alcool ou d'un alcoolat, par rapport au poids total de la composition.

3. Composition selon les revendications 1 ou 2, **caractérisée en ce qu'**il s'agit d'une composition pharmaceutique.

4. Composition selon les revendications 1 ou 2, **caractérisée en ce qu'**il s'agit d'une composition diététique.

5. Composition selon l'une quelconque des revendications 1 à 4, comprenant un seul sel de L-arginine.

6. Composition selon l'une quelconque des revendications 1 à 5, comprenant un poids d'eau égal à 1,8 fois à 2,5 fois le poids du sel d'arginine de la composition, ou un poids d'eau compris entre ces deux limites.

7. Composition selon l'une quelconque des revendications 1 à 6, comprenant en outre au moins un édulcorant fort.

8. Composition selon la revendication 7, dans laquelle ledit édulcorant fort est du sucralose et/ou de l'aspartame.

9. Composition selon la revendication 8, dans laquelle le ou les édulcorants fort(s), est (sont) présent(s) à raison, respectivement, de 0,01% à 1% en poids, de préférence de 0,01% à 0,5% en poids, par rapport au poids total de la composition.

10. Composition selon l'une quelconque des revendications 1 à 9, comprenant en outre au moins un additif conservateur et/ou au moins un antioxydant.

11. Composition selon l'une quelconque des revendications 1 à 10 comprenant au moins un sel de L-arginine à raison d'un gramme à dix grammes, de préférence cinq grammes ou huit grammes, par unité de prise.

12. Composition selon l'une quelconque des revendications 1 à 11, dans laquelle le sel d'arginine est choisi parmi l'aspartate d'arginine, le chlorhydrate d'arginine, le glutamate d'arginine, le pyroglutamate d'arginine, le pidolate d'arginine, le kétoglutarate d'arginine ou leurs mélanges.

13. Composition selon l'une quelconque des revendications 1 à 12, comprenant en outre au moins une purine, de préférence l'adénosine monophosphate (AMP), et/ou au moins un composé minéral ou un de ses sels, de préférence du magnésium, du potassium ou un de leurs sels, et/ou au moins un acide aminé autre que l'arginine, ou un métabolite dudit acide aminé ou un de leurs sels.

14. Composition selon l'une quelconque des revendications 1 à 13, comprenant en outre au moins une substance active autre qu'un sel de L-arginine choisie parmi la caféine, le guarana, la maca, une vitamine, et l'acide acétylsalicylique.

15. Composition selon l'une quelconque des revendications 1 à 14, **caractérisée en ce qu'**elle est sous une forme administrable par voie orale ou sublinguale, sous forme liquide, semi-liquide ou sous une forme lyophilisée préparée à partir d'une composition liquide ou semi-liquide.

16. Composition alimentaire liquide ou semi-liquide contenant au moins une composition diététique selon l'une quelconque des revendications 4 à 15.

17. Composition pharmaceutique ou diététique selon l'une quelconque des revendications 1 à 15, pour son utilisation
- dans la prévention et/ou le traitement des troubles de la réponse physiologique et/ou anatomique à la stimulation sexuelle chez les humains ;
- dans la prévention et/ou le traitement des affections cardiovasculaires dans lesquelles une relaxation des fibres musculaires lisses du cœur et des vaisseaux est souhaitée ou apporte un bénéfice, notamment l'hypertension artérielle, l'artérite des membres inférieurs, l'insuffisance cardiaque et les cardiopathies ischémiques ;
- dans la prévention et/ou le traitement des affections cardiovasculaires dues ou associées à un dysfonctionnement des cellules endothéliales vasculaires, par exemple, l'athérosclérose et/ou le diabète, ou encore conditionnées par l'âge ou l'intoxication tabagique ;
- dans la prévention et/ou le traitement d'une baisse de l'immunité ;
- dans la prévention et/ou le traitement des états de fatigue ; ou
- dans la stimulation de l'hormone de croissance.

18. Utilisation d'une association de maltitol, et d'un alcool ou d'un alcoolat, pour masquer l'amertume de l'arginine administrée sous forme salifiée dans une composition pharmaceutique, diététique ou alimentaire.

## Patentansprüche

1. Pharmazeutische oder diätetische, wässrige, flüssige oder halbflüssige Zusammensetzung, umfassend mindestens ein L-Arginin-Salz in einer Menge von mindestens einem Gramm des Salzes pro Dosierungseinheit, wobei die Zusammensetzung umfasst:
- Maltitol, bevorzugt in einer Menge des 1- bis 1,5-fachen Gewichts des Arginin-Salzes in der Zusammensetzung, und
- Wasser, in einer Gewichtsmenge des 1,5- bis 3-fachen Gewichts des Arginin-Salzes in der Zusammensetzung, und
- mindestens einen Alkohol oder ein Alkoholat.

2. Pharmazeutische oder diätetische, wässrige, flüssige oder halbflüssige Zusammensetzung, umfassend mindestens ein L-Arginin-Salz in einer Menge von mindestens einem Gramm des Salzes pro Dosierungseinheit, wobei die Zusammensetzung umfasst:
- 15 bis 30 Gewichtsprozent, bevorzugt 20 bis 30 Gewichtsprozent des L-Arginin-Salzes;
- 15 bis 45 Gewichtsprozent, bevorzugt 20 bis 40 Gewichtsprozent Maltitol, bezogen auf das Gesamtgewicht der Zusammensetzung, und
- 0,1 bis 12 Gewichtsprozent, bevorzugt 1 bis 5 Gewichtsprozent eines Alkohols oder eines Alkoholats, bezogen auf das Gesamtgewicht der Zusammensetzung.

3. Zusammensetzung nach Anspruch 1 oder 2, **dadurch gekennzeichnet, dass** es sich um eine pharmazeutische Zusammensetzung handelt.

4. Zusammensetzung nach Anspruch 1 oder 2, **dadurch gekennzeichnet, dass** es sich um eine diätetische Zusammensetzung handelt.

5. Zusammensetzung nach einem der Ansprüche 1 bis 4, umfassend ein einziges L-Arginin-Salz.

6. Zusammensetzung nach einem der Ansprüche 1 bis 5, umfassend ein Gewicht an Wasser, das gleich dem 1,8- bis 2,5-fachen des Gewichts des Arginin-Salzes der Zusammensetzung ist, oder ein Gewicht an Wasser, das zwischen diesen beiden Grenzwerten liegt.

7. Zusammensetzung nach einem der Ansprüche 1 bis 6, zudem umfassend mindestens ein starkes Süßungsmittel.

8. Zusammensetzung nach Anspruch 7, in welcher das starke Süßungsmittel Sucralose und/oder Aspartam ist.

9. Zusammensetzung nach Anspruch 8, in welcher das oder die starke(n) Süßungsmittel jeweils in einer Menge von 0,01 bis 1 Gewichtsprozent, bevorzugt von 0,01 bis 0,5 Gewichtsprozent, bezogen auf das Gesamtgewicht der Zusammensetzung, vorhanden ist (sind).

10. Zusammensetzung nach einem der Ansprüche 1 bis 9, zudem umfassend mindestens einen konservierenden Zusatzstoff und/oder mindestens ein Antioxidans.

11. Zusammensetzung nach einem der Ansprüche 1 bis 10, umfassend mindestens ein L-Arginin-Salz in einer Menge von einem Gramm bis zehn Gramm, bevorzugt fünf Gramm oder acht Gramm, pro Dosierungseinheit.

12. Zusammensetzung nach einem der Ansprüche 1 bis 11, in welcher das Arginin-Salz ausgewählt ist aus Argininaspartat, Argininhydrochlorid, Argininglutamat, Argininpyroglutamat, Argininpidolat, Argininketoglutarat oder deren Mischungen.

13. Zusammensetzung nach einem der Ansprüche 1 bis 12, zudem umfassend mindestens ein Purin, bevorzugt Adenosinmonophosphat (AMP) und/oder mindestens eine mineralische Verbindung oder eines ihrer Salze, bevorzugt Magnesium, Kalium oder eines ihrer Salze, und/oder mindestens eine weitere Aminosäure außer Arginin, oder einen Metabolit der Aminosäure oder eines ihrer Salze.

14. Zusammensetzung nach einem der Ansprüche 1 bis 13, zudem umfassend mindestens eine weitere aktive Substanz außer dem L-Arginin-Salz, welche ausgewählt ist aus Koffein, Guarana, Maca, einem Vitamin und Acetylsalicylsäure.

15. Zusammensetzung nach einem der Ansprüche 1 bis 14, **dadurch gekennzeichnet, dass** sie in einer auf oralem oder sublingualem Weg verabreichbaren Form, in flüssiger, halbflüssiger oder in einer aus einer flüssigen oder halbflüssigen Zusammensetzung hergestellten, lyophilisierten Form vorliegt.

16. Flüssige oder halbflüssige Nahrungsmittelzusammensetzung, enthaltend mindestens eine diätetische Zusammensetzung nach einem der Ansprüche 4 bis 15.

17. Pharmazeutische oder diätetische Zusammensetzung nach einem der Ansprüche 1 bis 15, zur Verwendung:
- in der Vorbeugung und/oder der Behandlung von Störungen der physiologischen und/oder anatomischen Antwort auf sexuelle Stimulation beim Menschen;
- in der Vorbeugung und/oder der Behandlung von Herz-Kreislauf-Erkrankungen, bei denen eine Entspannung der glatten Muskelfasern des Herzens und der Gefäße erwünscht ist oder einen Vorteil bringt, insbesondere arteriellem Bluthochdruck, Arteriitis der unteren Gliedmaßen, Herzinsuffizienz und ischämischen Herzkrankheiten;
- in der Vorbeugung und/oder der Behandlung von Herz-Kreislauf-Erkrankungen, die von einer Fehlfunktion der Gefäßendothelzellen verursacht oder begleitet werden, zum Beispiel Atherosklerose und/oder Diabetes, oder auch durch Alter oder Tabakmissbrauch bedingt sind;
- in der Vorbeugung und/oder der Behandlung einer Schwächung des Immunsystems;
- in der Vorbeugung und/oder der Behandlung von Erschöpfungszuständen; oder
- in der Stimulation des Wachstumshormons.

18. Verwendung einer Kombination aus Maltitol und einem Alkohol oder einem Alkoholat, um den bitteren Geschmack des in Form eines Salzes verabreichten Arginins in einer pharmazeutischen, diätetischen oder Nahrungsmittel-Zusammensetzung zu überdecken.

## Claims

1. A liquid or semi-liquid, aqueous dietary or pharmaceutical composition, comprising at least one salt of L-arginine in an amount of at least one gram of said salt per unit dose, said composition comprising:
- maltitol preferably in an amount of 1 to 1.5 times the weight of said arginine salt in the composition, and
- water, in an amount of 1.5 to 3 times the weight of said arginine salt in the composition, and
- at least one alcohol or alcoholate.

2. A liquid or semi-liquid, aqueous dietary or pharmaceutical composition, comprising at least one salt of L-arginine in an amount of at least one gram of said salt per unit dose, said composition comprising:
- from 15 to 30% by weight, preferably from 20% to 30% by weight of L-arginine salt;
- from 15 to 45%, preferably from 20% to 40% by weight of maltitol, relative to the total weight of the composition, and
- from 0.1 % to 12% by weight, preferably from 1 to 5% by weight of an alcohol or of an alcoholate, relative to the total weight of the composition.

3. A composition according to claims 1 or 2, **characterized in that** it is a pharmaceutical composition.

4. A composition according to claims 1 or 2, **characterized in that** it is a dietary composition.

5. A composition according to any one of claims 1 to 4, comprising a single salt of L-arginine.

6. A composition according to any one of claims 1 to 5, comprising a weight of water equal to a 1.8 times to 2.5 times the weight of arginine salt of the composition, or a weight of water comprised between these two limits.

7. A composition according to any one of claims 1 to 6, further comprising at least one strong sweetener.

8. A composition according to claim 7, wherein said strong sweetener is sucralose and/or aspartame.

9. A composition according to claim 8, wherein the strong sweetener(s) is (are) present in an amount, respectively, of from 0.01 % to 1 % by weight, preferably from 0.01 % to 0.5% by weight, relative to the total weight of the composition.

10. A composition according to any one of claims 1 to 9, further comprising at least one preservative additive and/or at least one antioxidant.

11. A composition according to any one of claims 1 to 10, comprising at least one L-arginine salt in an amount of one to ten grams, preferably five grams or eight grams, per unit dose.

12. A composition according to any one of claims 1 to 11, wherein the arginine salt is chosen from arginine aspartate, arginine hydrochloride, arginine glutamate, arginine pyroglutamate, arginine pidolate, arginine ketoglutarate, or mixtures of these.

13. A composition according to any one of claims 1 to 12, further comprising at least one purine, preferably adenosine monophosphate (AMP), and/or at least one mineral component or a salt thereof, preferably magnesium, potassium or one of their salts, and/or at least one amino acid other than arginine, or a metabolite of said amino acid or a salt thereof.

14. A composition according to any one of claims 1 to 13, further comprising at least one active ingredient other than a salt of L-arginine chosen from cafeine, guarana, maca, a vitamin, and acetylsalicylic acid.

15. A composition according to any one of claims 1 to 14, **characterized in that** it is in a form which may be taken orally or sublingually, in liquid, semi-liquid form or a lyophilized form prepared from a liquid or semi-liquid composition.

16. A liquid or semi-liquid food composition containing at least one dietary composition according to any one of claims 4 to 15,

17. A pharmaceutical or dietary composition according to any one of claims 1 to 15, for use
- in the prevention and/or the treatment of disorders of the physiological and/or anatomical response to sexual stimulation in humans;
- in the prevention and/or the treatment of the cardiovascular disorders in which a relaxation of the smooth muscle fibers of the heart and vessels is desired or provides benefit, for example such as high blood pressure, arteritis of the lower limbs, heart failure and ischemic heart diseases;
- in the prevention and/or the treatment of the cardiovascular disorders due to or associated with dysfunction of the vascular endothelial cells, for example atherosclerosis and/or diabetes, or for instance that are affected by age or tobacco addiction;
- in the prevention and/or the treatment of a reduction in immunity;
- in the prevention and/or the treatment of states of fatigue; or
- in the stimulation of growth hormone.

18. The use of an association of maltitol, and an alcohol or an alcoholate, to mask the bitterness of arginine taken in salified form in a food, dietary or pharmaceutical composition.
